# EUROPEAN PATENT SPECIFICATION

(11) **EP 2 801 262 B1**
(45) Date of publication and mention of the grant of the patent: **15.11.2017**
(21) Application number: 12864435.8
(22) Date of filing: 25.12.2012
(51) Int. Cl.: A23P 20/18, A23L 29/30, A23L 2/60, A61K 9/14, A23L 27/30

(54) **SUGAR COMPOSITION MAINLY COMPOSED OF SUCROSE AND D-PSICOSE, AND METHOD FOR PRODUCING SAME**
ZUCKERZUSAMMENSETZUNG HAUPTSÄCHLICH AUS SUCROSE UND D-PSICOSE SOWIE HERSTELLUNGSVERFAHREN DAFÜR
SUCRE COMPOSÉ PRINCIPALEMENT DE SACCHAROSE ET DE D-PSICOSE, ET SON PROCÉDÉ DE PRODUCTION

(30) Priority: 06.01.2012 JP 2012000898
(43) Date of publication of application: 12.11.2014
(73) Proprietor: Matsutani Chemical Industry Co., Ltd., Itami-shi, Hyogo 664-8508 (JP)
(72) Inventor: TAKAOKA, Seizo, Itami-shi Hyogo 664-8508 (JP); YAMADA, Takako, Itami-shi Hyogo 664-8508 (JP)
(74) Representative: TBK
(86) International application number: PCT/JP2012/083430
(87) International publication number: WO 2013/103106

(56) References cited:
- EP-A1- 1 864 669
- WO-A1-2008/059623
- JP-A- 2001 011 090
- JP-A- 2007 525 983
- US-A1- 2010 166 678
- Hisaka Oshima +ῃ ET AL: "Psicose Contents in Various Food Products and its Origin", , 24 April 2006 (2006-04-24), XP055167568, Retrieved from the Internet: URL:http://www.panelamonitor.org/media/doc repo/document/files/psicose-contents-in-va rious-food-products-and-its-origin.pdf [retrieved on 2015-02-05]

## Description

### Technical Field

The present invention relates to a sugar composition containing sucrose and D-psicose as main ingredients, and a manufacturing method and an application technique of the same.

### Background Art

D-psicose is one of rare sugars and it is produced from D-fructose in a yield of 20 to 25% by allowing D-ketohexose 3-epimerase (Patent Document 1) to act on D-fructose. Alternatively, there is a report that when D-psicose 3-epimerase (Non-Patent Document 1) is used, D-psicose is produced in a yield of 40%, and in the case of additionally using boric acid, D-psicose is produced in 62%. There is a report that, in the case of using an alkali isomerization method (Patent Document 2), D-psicose is produced in a yield of 10% or more from an isomerized sugar.

In addition, it has been revealed that D-psicose has characteristics as a lifestyle-related diseases prevention material, such as having no calories (Non-Patent Document 2), effect of suppressing postprandial blood glucose level (Non-Patent Document 3), and anti-obesity effect (Non-Patent Document 4).

Furthermore, D-psicose has a degree of sweetness of approximately 70% of that of sucrose, and when used alone as a sweetener, D-psicose is different from sucrose both in the degree and taste quality of sweetness.

WO 2005/084457 A1 describes an edible product comprising a core composition comprising sucrose and a shell or coating composition comprising at least one dietary fiber component, wherein the core composition is at least partially coated or encapsulated by the shell composition, a method of production of such an edible product, and its use.

WO 2005/103304 A1 is directed at a sugar substitute, in particular, a sweetener composition, wherein this composition comprises granules having a core including a nutritive sweetener and a layer on this core including sucralose

### Citation List

### Patent Documents

Patent Document 1: JP-A-H06-125776
Patent Document 2: WO2008/142860
Patent Document 3: WO2010/113785
Patent Document 4: JP-A-2007-091696

### Non-Patent Documents

Non-Patent Document 1: Am. J. Clin. Nutr., 2004, 79, 774-9
Non-Patent Document 2: J. Nutr. Sci. Vitaminol., 48, 77-80
Non-Patent Document 3: J. Nutr. Sci. Vitaminol., 59, 191-121
Non-Patent Document 4: J. Nutr. Sci. Vitaminol., 30, 55-65

### Summary of the Invention

### Problems that the Invention is to Solve

Sweeteners as typified by sucrose are not only used in liquid foods such as drinks, but also taken sometimes by dredging the sweetener over a surface of a cake in a solid state, or by dissolving the sweetener which is a solid or powder just before the consumption, such as a table sugar, in a coffee or another drink. D-psicose, which is a rare sugar, has physiological effects of, for example, lowering body fat, and a combination use of D-psicose with sucrose enables production of a sweetener having physiological effects such as prevention of obesity. However, it was found that there were problems that uniformity could not be maintained during storage when D-psicose crystals in a powder form were simply mixed with sucrose and that crystals having a constant composition could not be obtained from a mixture solution of sucrose and D-psicose. It is demanded to develop a homogeneous sugar composition containing sucrose and D-psicose as main ingredients.

An object of the present invention is to provide a homogeneous sugar composition containing as main ingredients sucrose, which is widely used as a sweetener in the food industry, and D-psicose, which has physiological effects such as lowering body fat.

More specifically, the object of the invention is to provide a sweetener which has a degree of sweetness and a taste quality very close to those of superfine sugar and to produce the sweetener by homogeneous dispersion-coating of D-psicose onto sucrose, which has been widely used as a sweetener in the food industry.

In addition, another object of the invention is to provide a novel sweetener which overcomes problems of sucrose, which is widely used as a sweetener in the food industry, such as causing lifestyle diseases like obesity.

**Moreover, still another object of the invention is to provide the use of a sucrose mixture that contains D-psicose in a food or drink, a drug or quasi-drug, or a cosmetic.**

### Means for Solving the Problems

The present inventors have repeated an extensive and intensive research and development in order to solve the above problems. Although an effect of preventing obesity can be achieved without impairing the taste of the sucrose by mixing sucrose and D-psicose in a certain ratio, when considering the provision in a powder form for use as table sugar or the like, a simple powder mixture of sucrose and D-psicose becomes heterogeneous due to tapping or the like during storage and is not applicable for practical use. Co-crystal of sucrose and D-psicose is also possibly proposed, but, in general, the process is troublesome, and in the superfine sugar, hydrolysis and condensation of sucrose and D-psicose may occur under a condition of high temperature for satisfying the condition for precipitation, thereby causing caramel color. It is also difficult to produce co-crystals having a constant composition. The present inventors found that a homogeneous coating composition can be obtained by adding a supersaturated solution of D-psicose to sucrose crystals to deposit D-psicose in a crystallized state or an amorphous state together with the sucrose crystals, and thus the present invention has been successfully completed.

Specifically, the present invention provides a homogeneous sugar composition containing sucrose and D-psicose as main ingredients, which is intended to have an effect of physiological activity of D-psicose.

The gist of the present invention includes the sugar composition and its use as set forth in (1) to (6) below and the manufacturing method thereof as set forth in (7) below.
(1) A sugar composition, characterized by containing sucrose and D-psicose, wherein crystals of the sucrose are coated with the D-psicose in a crystalline or amorphous state wherein the sugar composition is obtainable as defined in claim 1.
(2) The sugar composition according to the above (1), wherein the content of the D-psicose is 1 part by weight or more and 50 parts by weight or less relative to 100 parts by weight of the total weight of the sucrose and the D-psicose.
(3) The sugar composition according to the above (1) or (2), **wherein the composition contains 3 parts by weight or more of D-psicose therein.**
(4) The sugar composition according to the above (1), (2), or (3), wherein the D-psicose is produced by an alkali isomerization method for isomerizing glucose and fructose with an alkali or by an enzyme method for isomerizing glucose and fructose by allowing an enzyme to act thereon, or the D-psicose is produced bv an extraction from a plant containing D-psicose.
(5) **Use of the sugar composition** according to any one of the above (1) to (4), **for the preparation of** a food or drink, a drug or quasi-drug, or a cosmetic.
(6) **Use of the sugar composition** according to any one of the above (1) to (4), **as** a sweetener having a degree of sweetness and a quality of taste.
**(7)** A method for manufacturing the sugar composition
   **according to any one of the above 4**, comprising the following (a) or (b) :
   (a) mixing sucrose crystals with a supersaturated solution of D-psicose which has been heated in a range of 90 to 50°C in advance, stirring the mixture while gradually lowering the temperature to promote crystallization of the D-psicose, thereby coating the sucrose crystals with the D-psicose in a crystalline or amorphous state;
   (b) dissolving sucrose and D-psicose, crystallizing the sucrose first, and thereafter coating the sucrose crystals with the D-psicose in a crystalline form.

### Advantage of the Invention

According to the present invention, it is possible to provide a homogeneous sugar composition containing as main ingredients sucrose, which is widely used as a sweetener in the food industry, and D-psicose, which has physiological effects such as lowering body fat. More specifically, according to the present invention, it is possible to provide a sweetener having a degree of sweetness and a quality of taste very close to those of a superfine sugar, the sweetener being produced by homogeneously dispersion-coating sucrose, which is widely used as a sweetener in the food industry, with D-psicose.

In addition, this invention can provide a novel sweetener which overcomes problems of sucrose, which is widely used as a sweetener in the food industry, such as causing lifestyle diseases like obesity.

**Furthermore, this invention can provide the use of a D-psicose in a food or a drink**, a drug or quasi-drug, or a cosmetic.

### Mode for Carrying Out the Invention

### [Sucrose]

Sucrose, as used in the invention, is referred to as a sugar mainly containing sucrose and commonly referred to as sucrose, such as a superfine sugar, a powder sugar, a brown sugar, and Wasanbon (refined Japanese sugar).

### [D-Psicose]

D-Psicose, as used in the invention, is a sugar which has a "history of use in food" (history of being eaten by a common people) in spite of being a rare sugar of monosaccharide present in the natural world in only a small amount. The sweetness thereof is 70% of that of sucrose, and the calorie content thereof is almost zero. D-psicose has physiological effects such as suppression of postprandial glucose increase, and reduction of body fat. The D-psicose may be obtained by any means, for examples extracting from the natural world, and synthesizing with a chemical or a biological method. As a relatively easy procedure, the D-psicose may be prepared, for example, by a method of extracting it from natural Itea plants or the like, a method of allowing 3-epimerase to act on D-fructose (for example, Patent Document 2), or a method of isomerizing hexose under an alkali condition (Patent Document 3).

### [Sugar composition]

A concrete aspect of the sugar composition of the invention is a sugar composition having a degree of sweetness and a quality of taste equal to those of sucrose, in which sucrose crystals are uniformly coated with D-psicose in a crystalline form or in an amorphous state, and the content of the D-psicose is 1 to 50 parts by weight, preferably 1 to 15 parts by weight relative to 100 parts by weight of the total weight of the sucrose and the D-psicose. Such a sugar composition is intended to have an effect of physiological activity of D-psicose attained by containing 3 parts by weight or more therein.

### [Manufacturing method of sugar composition]

The method for manufacturing the sugar composition of the invention will be described.

Upon manufacturing the sugar composition of the invention, the particle diameter of the crystals of the sucrose to be used is not specified, but the particle diameter of the raw material sucrose crystals is reflected in the particle diameter of the final sweetener. The particle diameter of the raw material sugar crystals is appropriately selected according to the particle diameter of the intended sugar composition. The final sweetener may also be pulverized into a smaller particle diameter. Sieving may be employed, as required. Sucrose can be coated with D-psicose homogenously in the final sugar composition, and therefore it has become possible to supply the final sugar composition without being classified by the transport or vibration.

As for a method for coating sucrose with D-psicose, a supersaturated solution of D-psicose that has been heated in advance is mixed with sucrose crystals, the mixture is stirred while gradually lowering the temperature to promote the crystallization of the D-psicose. This procedure is continuously carried out, whereby a crystal surface of the sucrose is coated with the D-psicose in a crystalline or amorphous state or mixed crystals with the sucrose crystals are produced. In this procedure, the temperature of the supersaturated solution of D-psicose to be heated is preferably approximately 90 to 50°C, and more preferably 70°C. At a lower temperature, the concentration of the supersaturated solution is lowered, whereas at an excessively high temperature, possibility of browning of the solution is higher. Alternatively, it is possible that sucrose and D-psicose are dissolved and the sucrose is allowed to be crystallized first, and thereafter the sucrose is coated with D-psicose in a crystalline form.

According to the present invention, it has become possible to provide functions (postprandial blood glucose suppressing effect, anti-obesity effect, etc.) of D-psicose to sucrose, which is widely used as a sweetener in the food industry and in drugs and quasi-drugs, and cosmetics.

Accordingly, the sugar composition of the invention includes products to which a sweet taste is imparted, such as a food or drink, a drug or quasi-drug, and a cosmetic, to which a sweet taste is imparted.

The food or drink referred to in this invention means a general sense of food product which requires a sweet taste, such as a drink, candy, frozen dessert, yogurt, and chocolate. The other products to which a sweet taste is imparted include products of a drug, a quasi-drug such as an oral composition, and a cosmetic, to which a sweet taste is imparted.

The sugar composition of the invention contains D-psicose and has an obesity preventing effect.

In addition, the sugar composition may be used in a combination or a mixture with other active ingredients for suppressing postprandial blood glucose.

The sugar composition of the invention can be used in any products that require a sweet taste, such as a food, a health food, a patient food, a food material, a health food material, a patient food material, a food additive, a health food additive, a patient food additive, a drink, a health drink, a patient drink, a drinking water, a health drinking water, a patient drinking water, a drug, a pharmaceutical material, a feedstuff, and a feedstuff for a patient domestic animal and/or a patient wild animal.

When the sugar composition of the invention is used in a food product, the composition may be used as it is or in a form of a suspension in oil or the like.

Examples of the usage form of the sugar composition (sweetener) of the invention include tablet and capsule when it is used as a food material or a food additive intended to improve abnormal carbohydrate metabolism and/or abnormal lipid metabolism.

In addition, the sugar composition (sweetener) of the invention may be appropriately added to a food product to prepare a health food or a food for sick people intending to suppress postprandial blood glucose and/or to prevent obesity. As an optional ingredient, a vitamin, a carbohydrate, a coloring matter, or a flavor which are commonly added to a food product may be appropriately blended therein. The food product may be taken in any form of liquid or solid. The food product may be encapsulated in gelatin or the like and taken as a soft capsule agent. The capsule may be formed of a gelatin coating which is prepared, for example, by adding water to a raw material gelatin to dissolve the gelatin in water, and adding a plasticizer (glycerol, D-sorbitol, etc.) thereto.

The sugar composition (sweetener) of the invention may be used for the same purpose as in sucrose. The sugar composition may be used for cooking, or in tea, coffee, seasoning (Mirin (Japanese sweet seasoning), etc.), or the like.

Furthermore, the sugar composition may be added to a tooth paste or the like for use as a sweetener.

A soluble film is becoming used for formulation of a cosmetic or the like. For example, edible soluble film is used as a flavor film or the like in which a flavor or the like is retained for the purpose of refreshment, bad breath prevention, or the like. There is proposed a soluble film (Patent Document 4) which has a good solubility and good film characteristics as a packaging material of a food product, a drug, etc., or as a carrier for retaining an active ingredient of a food product, a drug, etc., and can be suitably used in such an application. As such, the sweetener of the invention having a degree of sweetness and a quality of taste of sucrose can be applied in a drug or quasi-drug, or a cosmetic.

The present invention will be described in detail below with reference to examples, but the invention is not to be limited to these examples.

### Example 1

Into a stirrer-equipped stainless container (cylindrical shape) in which the temperature can be controlled and the pressure can be reduced, 190 g of a commercially available sucrose (manufactured by Mitsui Sugar, Co., Ltd.) was weighed, a solution of D-psicose (manufactured by Izumoring Co., Ltd.) (D-psicose concentration: 1.2 times the saturation concentration at 70°C) which had been heated to 70°C was added thereto with stirring in an amount corresponding to 10 g in terms of the solid content, and the mixture was dried while gradually reducing the pressure. (The final D-psicose content was to be 5%.)

### Example 2

Into a stirrer-equipped stainless container (cylindrical shape) in which the temperature can be controlled and the pressure can be reduced, 180 g of a commercially available sucrose (manufactured by Mitsui Sugar Co. , Ltd.) was weighed, a solution of D-psicose (manufactured by Izumoring Co., Ltd.) (D-psicose concentration: 1.2 times the saturation concentration at 70°C) which had been heated to 70°C was added thereto with stirring in an amount corresponding to 20 g in terms of the solid content, and the mixture was dried while gradually reducing the pressure. (The final D-psicose content was 10%.)

### Example 3

Into a stirrer-equipped stainless container (cylindrical shape) in which the temperature can be controlled and the pressure can be reduced, 170 g of a commercially available sucrose (manufactured by Mitsui Sugar Co. , Ltd.) was weighed, a solution of D-psicose (manufactured by Izumoring Co., Ltd.) (D-psicose concentration: 1.2 times the saturation concentration at 70°C) which had been heated to 70°C was added thereto with stirring in an amount corresponding to 30 g in terms of the solid content, and the mixture was dried while gradually reducing the pressure. (The final D-psicose content was 15%.)

### Example 4

Into a stirrer-equipped stainless container (cylindrical shape) in which the temperature can be controlled and the pressure can be reduced, 160 g of a commercially available sucrose (manufactured by Mitsui Sugar Co. , Ltd.) was weighed, a solution of D-psicose (manufactured by Izumoring Co., Ltd.) (D-psicose concentration: 1.2 times the saturation concentration at 70°C) which had been heated to 70°C was added thereto with stirring in an amount corresponding to 40 g in terms of the solid content, and the mixture was dried while gradually reducing the pressure. (The final D-psicose content was 20%.)

### Example 5

Into a stirrer-equipped stainless container (cylindrical shape) in which the temperature can be controlled and the pressure can be reduced, 100 g of a commercially available sucrose (manufactured by Mitsui Sugar Co. , Ltd.) was weighed, a solution of D-psicose (manufactured by Izumoring Co., Ltd.) (D-psicose concentration: 1.2 times the saturation concentration at 70°C) which had been heated to 70°C was added thereto with stirring in an amount corresponding to 100 g in terms of the solid content, and the mixture was dried while gradually reducing the pressure. (The final D-psicose content was 50%.)

### Example 6

About 50 g of each of the compositions prepared in Examples 1, 2, 3, 4, and 5 and a commercially available sucrose (manufactured by Mitsui Sugar Co., Ltd.) was weighed into a petri dish, and while allowing the petri dish to stand under an environment at a room temperature of 20°C and with a humidity uncontrolled, the state was observed for 15 days. The results are shown in Table 1.

**[Table 1]**

| | After 1 day | After 3 days | After 5 days | After 10 days | After 15 days |
|---|---|---|---|---|---|
| Example 1 | A | A | A | B | B |
| Example 2 | A | A | A | B | B |
| Example 3 | A | A | A | B | B |
| Example 4 | A | A | B | B | C |
| Example 5 | A | A | B | C | D |
| Sucrose | A | A | A | B | B |

| | | | | | |
|---|---|---|---|---|---|
| A = No change, B = Caking a little, C = Caking, D = Deliquescing | | | | | |

As found in Table 1, the sugar had no problem in the deliquescence as compared with the commercially available sucrose until the addition level of 15% in D-psicose content. Also at the addition level of 50% in D-psicose content, the sugar had no problem until 5 days, and when the sugar was sealed in a container, deliquescence thereof was not observed even after 15 days. It was considered that the sugar was usable by sealing in use.

### Example 7

Into a petri dish, 100 g of each of the composition prepared in Example 1 and a commercially available sucrose (manufactured by Mitsui Sugar Co., Ltd.) was weighed, the dish was stored in a thermo-hygrostat of RH52 for 20 days, and the properties and the weight increase were determined. The results are shown in Table 2.

**[Table 2]**

| | Properties | Weight increase |
|---|---|---|
| Composition of Example 1 | No change | 0.24 g |
| Commercially available sucrose | No change | 0.19 g |

As shown in Table 2, the composition obtained in Example 1 was at an equal level with the commercially available sucrose.

### Example 8

In a plastic cup, 90% of sucrose crystals (granulated sugar manufactured by Mitsui Sugar Co., Ltd.) and 10% of D-psicose (D-psicose crystals manufactured by Izumoring Co., Ltd.) were placed in a total weight of 500 g, and mixed by sufficient stirring for 10 minutes, and the mixture was charged into a standing pouch (Manufactured by Dainippon Seisan-sha), and the pouch was set on a Ro-tap shaker and shaken for 20 minutes. Four samples were taken from upper, lower, right and left portions and the sugar composition (the ratio of sucrose and D-psicose) of each sample was determined by a high-performance liquid chromatography. The results are shown in Table 3.

**[Table 3]**

| | After mixing Upper | After mixing Middle | After mixing Lower | After shaking Upper right | After shaking Lower right | After shaking Upper left | After shaking Lower left |
|---|---|---|---|---|---|---|---|
| Sucrose: D-psicose | 91.8: 8.2 | 82.7: 17.3 | 87.9: 12.1 | 81.6: 18.4 | 72.3: 27.7 | 88.5: 11.5 | 78.0: 22.0 |

As shown in Table 3, it was found that sucrose crystals and D-psicose crystals could not be mixed completely only by powder mixing.

### Example 9

In order to compare the composition prepared in Example 1 with Example 8, 500 g of the composition was weighed and placed into a plastic cup, mixed by sufficient stirring for 10 minutes, charged into a standing pouch (Manufactured by Dainippon Seisan-sha), and the pouch was set on a Ro-tap shaker and shaken for 20 minutes. Four samples were taken from upper, lower, right and left portions and the sugar composition (the ratio of sucrose and D-psicose) of each sample was determined by a high-performance liquid chromatography. The results are shown in Table 4.

**[Table 4]**

| | After mixing Lower right | After mixing Upper right | After mixing Lower left | After mixing Upper left | After shaking Lower right | After shaking Upper right | After shaking Lower left | After shaking Upper left |
|---|---|---|---|---|---|---|---|---|
| Sucrose: D-psicose | 95.1:4.9 | 95.1:4.9 | 95.1:4.9 | 95.1:4.9 | 95.0:5.0 | 95.0:5.0 | 95.0:5.0 | 95.0:5.0 |

As shown in Table 4, the composition obtained in Example 1 was constituted at a uniform ratio, and the ratio did not change even though vibration was applied.

### Example 10

Five percent by weight of D-psicose powder (manufactured by Izumoring Co., Ltd.) was mixed with a commercially available sucrose (Mitsui Sugar Co., Ltd.), and a solution was prepared such that the concentration of the solution was 1.2 times the saturated concentration of D-psicose at 70°C, and 1 to 10% of a mixture of sucrose and D-psicose relative to the solid content in weight of the solution was added thereto as seed crystals. The resulting mixture was stirred while lowering the temperature of the solution to 60 to 20°C, and at the same time, the solution was dried under reduced pressure such that the concentration of the solution became 1.2 times the saturated concentration of D-psicose at 60 to 20°C, thereby promoting precipitation.

The crystals obtained were separated from the solution, and the crystals and the solution were tested according to a method described later. As a result, it was found that the crystals were sucrose alone and D-psicose remained in the solution.

### Example 11

The composition in which sucrose was coated with D-psicose (the ratio of sucrose and D-psicose: 62:3) was tested using rats with respect to the effects thereof on the body weight increase and the body fat accumulation.

### [Experimental method]

As the experimental animals, 3 week-old male Sprague-Dawley rats were used. The feed composition is shown in Table 5. As for the groups, a sucrose group, and a sweetener of the invention (sucrose + psicose) group were employed. The rats were raised under free feeding with the experimental feed (Table 5) and water for 4 weeks. The body weights were measured every week. The results are shown in Table 6.

After the raising, anatomy was performed. The body fat was taken during the anatomy, and the weight thereof was measured. The results are shown in Table 7.

**[Table 5]**

| | Sucrose | Sucrose + Psicose |
|---|---|---|
| Casein | 200 | 200 |
| Sucrose | 650 | - |
| Sweetener of the Invention | - | 650 |
| Cellulose | 50 | 50 |
| Mineral mixture (AIN-76) | 35 | 35 |
| Vitamin mixture (AIN-76) | 10 | 10 |
| DL-Methionine | 3 | 3 |
| Choline bitartrate | 2 | 2 |
| Cone oil | 50 | 50 |

**[Table 6]**

| Change in Body Weight | | | | | |
|---|---|---|---|---|---|
| Age of rat in weeks | 4 weeks | 5 weeks | 6-weeks | 7 weeks | 8 weeks |
| Test period | Initial | After 1 week | After 2 weeks | After 3 weeks | After 4 weeks |
| Sucrose | 90.0±2.7 | 145.6±3.4 | 199.1±2.3 | 271.0±2.8 | 320.6±2.1 |
| Sucrose + Psicose | 90.0±3.2 | 142.5±3.5 | 191.8±4.7 | 253.9±5.5 | 291.2±6.7 |

The numerical values represent (average value ± standard error) (g).

Comparison of Body Fat Value

| | Body fat tissue |
|---|---|
| Sucrose | 15.7±0.9 |
| Sucrose + Psicose | 9.1±0.7 |

The numerical values represent (average value ± standard error) (g).

### [With respect to the Results]

The average body weights of the Sprague-Dawley rats at the time of the anatomy were 320.6 g in the sucrose group, and 291.2 g in the (sucrose + psicose) group, and reductions in the body weight increase in the (sucrose + psicose) group were observed in all the weeks. As for the body fat weight, reduction in the weight was recognized more in the sweetener of the invention (sucrose + psicose) group compared to the sucrose group.

As is apparent from the above examples, a food or drink containing the composition of the present invention had an effect of improving body weight increase and/or body fat accumulation.

### Industrial Applicability

According to the present invention, sucrose is coated with D-psicose homogeneously, whereby it became possible to supply the composition without being classified by transport or vibration.

In addition, according to the invention, it became possible to provide functions of D-psicose to sucrose, which is widely used as a sweetener in the food industry and in drugs, quasi-drugs, and cosmetics.

## Claims

1. A sugar composition, **characterized by** containing sucrose and D-psicose, wherein crystals of the sucrose are coated with the D-psicose in a crystalline or amorphous state, wherein the sugar composition is obtainable by the following (1) or (2):
(1) mixing sucrose crystals with a supersaturated solution of D-psicose which has been heated in a range of 90 to 50°C in advance, stirring the mixture while gradually lowering the temperature to promote crystallization of the D-psicose, thereby coating the sucrose crystals with the D-psicose in a crystalline or amorphous state;
(2) dissolving sucrose and D-psicose, crystallizing the sucrose first, and thereafter coating the sucrose crystals with the D-psicose in a crystalline form.

2. The sugar composition according to claim 1, wherein the content of the D-psicose is 1 part by weight or more and 50 parts by weight or less relative to 100 parts by weight of the total weight of the sucrose and the D-psicose.

3. The sugar composition according to claim 1 or 2, wherein the composition contains 3 parts by weight or more of D-psicose therein.

4. The sugar composition according to claim 1, 2, or 3, wherein the D-psicose is produced by an alkali isomerization method for isomerizing glucose and fructose with an alkali or by an enzyme method for isomerizing glucose and fructose by allowing an enzyme to act thereon, or the D-psicose is produced by an extraction from a plant containing D-psicose.

5. Use of the sugar composition according to any one of claims 1 to 4, for the preparation of a food or drink, a drug or quasi-drug, or a cosmetic.

6. Use of the sugar composition according to any one of claims 1 to 4, as a sweetener.

7. A method for manufacturing the sugar composition according to any one of claims 1 to 4, comprising the following (1) or (2):
(1) mixing sucrose crystals with a supersaturated solution of D-psicose which has been heated in a range of 90 to 50°C in advance, stirring the mixture while gradually lowering the temperature to promote crystallization of the D-psicose, thereby coating the sucrose crystals with the D-psicose in a crystalline or amorphous state;
(2) dissolving sucrose and D-psicose, crystallizing the sucrose first, and thereafter coating the sucrose crystals with the D-psicose in a crystalline form.

## Patentansprüche

1. Zuckerzusammensetzung, **dadurch gekennzeichnet, dass** sie Saccharose und D-Psicose enthält, wobei Kristalle der Saccharose mit der D-Psicose in einem kristallinen oder amorphen Zustand überzogen sind, wobei die Zuckerzusammensetzung durch das Folgende (1) oder (2) erhältlich ist:
(1) Mischen von Saccharosekristallen mit einer übersättigten Lösung von D-Psicose, welche vorher in einem Bereich von 90 bis 50°C erwärmt wurde, Rühren der Mischung, während schrittweise die Temperatur gesenkt wird, um die Kristallisation der D-Psicose zu fördern, dadurch Überziehen der Saccharosekristalle mit der D-Psicose in einem kristallinen oder amorphen Zustand;
(2) Lösen von Saccharose und D-Psicose, zuerst Kristallisieren der Saccharose und danach Überziehen der Saccharosekristalle mit der D-Psicose in einer kristallinen Form.

2. Zuckerzusammensetzung nach Anspruch 1, wobei der Gehalt der D-Psicose relativ zu 100 Gewichtteilen des Gesamtgewichts der Saccharose und der D-Psicose 1 Gewichtsteil oder mehr und 50 Gewichtsteile oder weniger ist.

3. Zuckerzusammensetzung nach Anspruch 1 oder 2, wobei die Zusammensetzung 3 Gewichtsteile oder mehr D-Psicose darin enthält.

4. Zuckerzusammensetzung nach Anspruch 1, 2 oder 3, wobei die D-Psicose durch ein alkalisches Isomerisationsverfahren für die Isomerisierung von Glukose und Fruktose mit einer Base, oder durch ein Enzymverfahren für die Isomerisierung von Glukose und Fruktose durch Ermöglichen der Einwirkung eines Enzyms darauf, hergestellt wird, oder die D-Psicose durch eine Extraktion aus einer Pflanze, die D-Psicose enthält, hergestellt wird.

5. Verwendung der Zuckerzusammensetzung nach einem der Ansprüche 1 bis 4 für die Zubereitung eines Lebensmittels oder eines Getränks, eines Wirkstoffs oder Quasi-Wirkstoffs oder eines Kosmetikums.

6. Verwendung der Zuckerzusammensetzung nach einem der Ansprüche 1 bis 4 als ein Süßstoff.

7. Verfahren für die Herstellung der Zuckerzusammensetzung nach einem der Ansprüche 1 bis 4, das das Folgende (1) oder (2) umfasst:
(1) Mischen von Saccharosekristallen mit einer übersättigten Lösung von D-Psicose, welche vorher in einem Bereich von 90 bis 50°C erwärmt wurde, Rühren der Mischung, während schrittweise die Temperatur gesenkt wird, um die Kristallisation der D-Psicose zu fördern, dadurch Überziehen der Saccharosekristalle mit der D-Psicose in einem kristallinen oder amorphen Zustand;
(2) Lösen von Saccharose und D-Psicose, zuerst Kristallisieren der Saccharose und danach Überziehen der Saccharosekristalle mit der D-Psicose in einer kristallinen Form.

## Revendications

1. Composition de sucre, **caractérisée en ce qu'**elle contient du saccharose et du D-psicose, dans laquelle les cristaux du saccharose sont enrobés de D-psicose à l'état cristallin ou amorphe, où la composition de sucre peut être obtenue par l'opération (1) ou (2) suivante :
(1) mélange des cristaux de saccharose avec une solution sursaturée de D-psicose qui a été chauffée dans une plage de 90 à 50 °C au préalable, agitation du mélange tout en abaissant progressivement la température pour favoriser la cristallisation du D-psicose, pour enrober ainsi les cristaux de saccharose dans le D-psicose à l'état cristallin ou amorphe ;
(2) dissolution du saccharose et du D-psicose, cristallisation du saccharose en premier, puis enrobage des cristaux de saccharose avec le D-psicose sous une forme cristalline.

2. Composition de sucre selon la revendication 1, dans laquelle la teneur en D-psicose est de 1 partie en poids ou plus et de 50 parties en poids ou moins pour 100 parties en poids du poids total du saccharose et du D-psicose.

3. Composition de sucre selon la revendication 1 ou 2, dans laquelle la composition contient 3 parties en poids ou plus de D-psicose.

4. Composition de sucre selon la revendication 1, 2, ou 3, dans laquelle le D-psicose est produit par un procédé d'isomérisation alcalin pour l'isomérisation du glucose et du fructose avec un alcali ou par un procédé enzymatique pour l'isomérisation du glucose et du fructose en laissant une enzyme agir sur ceux-ci, ou le D-psicose est produit par extraction à partir d'une plante contenant du D-psicose.

5. Utilisation de la composition de sucre selon l'une quelconque des revendications 1 à 4, pour la préparation d'un aliment ou d'une boisson, d'un médicament ou quasi-médicament, ou d'un produit cosmétique.

6. Utilisation de la composition de sucre selon l'une quelconque des revendications 1 à 4, à titre d'édulcorant.

7. Procédé de fabrication de la composition de sucre selon l'une quelconque des revendications 1 à 4, comprenant l'opération (1) ou (2) suivante :
(1) mélange des cristaux de saccharose avec une solution sursaturée de D-psicose qui a été chauffée dans une plage de 90 à 50 °C au préalable, agitation du mélange tout en abaissant progressivement la température pour favoriser la cristallisation du D-psicose, pour enrober ainsi les cristaux de saccharose dans le D-psicose à l'état cristallin ou amorphe ;
(2) dissolution du saccharose et du D-psicose, cristallisation du saccharose en premier, puis enrobage des cristaux de saccharose dans le D-psicose sous une forme cristalline.
